# Europäisches Patentamt
# European Patent Office
# Office européen des brevets

(19)

(11) Publication number: **0 411 605 B1**

# EUROPEAN PATENT SPECIFICATION

(12)

(45) Date of publication of patent specification: **07.06.95**  (51) Int. Cl.⁶: **A61M 25/00**

(21) Application number: **90114762.9**

(22) Date of filing: **01.08.90**

(54) **Catheter and assembly for extracorporeal circulation.**

(30) Priority: **04.08.89 JP 201197/89**
**05.09.89 JP 228220/89**

(43) Date of publication of application:
**06.02.91 Bulletin 91/06**

(45) Publication of the grant of the patent:
**07.06.95 Bulletin 95/23**

(84) Designated Contracting States:
**BE DE FR GB IT NL SE**

(56) References cited:
**EP-A- 0 212 159      FR-A- 2 565 491**
**GB-A- 2 065 479      US-A- 3 547 119**
**US-A- 3 565 078      US-A- 4 173 981**

**THE ANNALS OF THORACIC SURPERY, vol. 336, no. 2, August 1983 PHILLIPS et al."Percutaneous Initiation of Cardiopulmonary Bypass" pages 223-225**

(73) Proprietor: **TERUMO KABUSHIKI KAISHA**
**No. 44-1, Hatagaya 2-chome,**
**Shibuya-ku**
**Tokyo 151 (JP)**

(72) Inventor: **Oshiyama, Hiroaki, c/o Terumo K.K.**
**1500 Inokuchi,**
**Nakai-cho**
**Ashigarakami-gun,**
**Kanagawa (JP)**
Inventor: **Sagae, Kyuta, c/o Terumo K.K.**
**1500 Inokuchi,**
**Nakai-cho**
**Ashigarakami-gun,**
**Kanagawa (JP)**

(74) Representative: **Casalonga, Axel et al**
**BUREAU D.A. CASALONGA - JOSSE**
**Morassistrasse 8**
**D-80469 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

## Description

The present invention relates to a catheter assembly for extracorporeal circulation which is suitable for use in heart treatment and the like.

Patients suffering from heart failure, e.g. myocardial infarction, have hitherto been treated by various methods such as percutaneous transluminal coronary angioplasty (hereinafter abbreviated to "PTCA"), and Intra Aortic Baloon Pumping (hereinafter abbreviated to "IABP").

In PTCA, a balloon is inserted into the constricted lesion of the coronary artery, and is then inflated to dilate the constricted lesion.

In IABP, a balloon is inserted in the base portion of the aorta, and is then repeatedly inflated and deflated synchronization with the heart beat to increase blood flow in the coronary artery, hence, to assist a heart whose function has degraded.

The conventional methods involve the following problems:
PTCA treatment may put a patient into a shocked condition, which is very dangerous; and IABP treatment can increase blood flow only to a limited extent because the heart assisting function can be degraded with a drop in the cardiac output, sometimes failing to assure the patient's recovery.

Because of those problems, it is sometimes necessary for heart treatment to be performed simultaneously with auxiliary circulation of the blood, wherein an extracorporeal blood circulation circuit is formed for this purpose during the heart treatment.

During the treatment of heart failure, e.g. acute myocardial infarction, because the heart function is degraded, emergency auxiliary circulation of blood is performed to add oxygen and remove carbon dioxide.

In such cases where auxiliary blood circulation is necessary, there is too little time to form the circuit by open heart surgery.

In such cases, therefore, catheters capable of percutaneous insertion are employed to allow blood to be drawn from and pumped back into the patient's body through the catheters, thereby effecting extracorporeal circulation. For instance, the catheter on the blood-drawing side of the circuit is inserted into the femoral vein by an insertion method, described later, with the distal end of the catheter reaching through the bifurcation of the vena cava to the right atrium and being indwelt therein.

However, with a conventional catheter device, since percutaneous insertion is conducted, the diameter of a catheter is inevitably small, thereby involving a great resistance to the flow of blood, hence, a great pressure loss. Thus, it is difficult to assure sufficient amounts of drawn-out blood and pumped-in blood.

When extracorporeal circulation is conducted simultaneously with the execution of PTCA or IABP heart treatment, in order to transfer oxygenated blood from a lung apparatus into the blood vessel, a blood-transfusion catheter device must be inserted into a blood vessel portion which is different from the portion where a balloon is inserted for the PTCA or IABP treatment. Thus, the insertion of the blood-transfusion catheter device makes the entire operation complicated, and creates an additional burden on the patient.

An object of the present invention is therefore to provide a catheter assembly with a catheter that has a diameter small enough to facilitate percutaneous insertion at the time of auxiliary blood circulation but is capable of assuring sufficient amounts of drawn-out and pumped-in blood.

US-A-3 547 119 (HALL) shows a catheter for peritoneal dialysis having lateral apertures in its end portion without giving precise dimensions of the side holes. The disclosed catheter is also permanently equipped with a valve assembly, so that it is impossible to introduce a dilator or other means in the catheter through a connecting instrument.

US-A-3 565 078 (VAILLIANCOURT) discloses a catheter coupling assembly having a valve liner adapted to receive a connector for introducing into the open end of a catheter. No indication is given on the structure of the catheter itself.

The article of Philipps et al "Percutaneous initiation of cardiopulmonary Bypass" in the Annales of Thoracic Surgery 1983 - pages 223-225 only shows the use of a cannula having a plurality of side holes as well as an end hole without giving an indication on the specific location of the side holes or the length of active parts of the cannula.

Another object of the present invention is to provide an assembly for extracorporeal circulation that enables extracorporeal circulation to be effected simultaneously with the execution of heart treatment.

As claimed, the catheter assembly of the invention comprises a catheter and a connecting instrument. The catheter has a hollow body introducible into a blood vessel and provided with an opening at a distal end thereof and a plurality of side holes, a proximal end communicating with the hollow inside of said body and having an introduction passage with a hemostatic valve disposed therein.

According to the invention, the area S1 of the opening at the distal end of said body is such as compared to the total opening area S2 of said plurality of side holes that the relation $2 \times S1 \geq S2 \geq 0.5 \times S1$ is satisfied.

The connecting instrument has a tubular portion adapted to be inserted in said introduction passage in a liquid-tight manner through the hemostatic valve, the inner surface of the tubular portion being tapered and the distal end of the tubular portion being disposed close or in abutting relation to the inner surface of the catheter body.

In an advantageous embodiment, the distance L1 from the distal end of said body to the side hole closest to said proximal end is such as compared to the whole length L2 of said body, that the relation of 2/3 X L2 ≧ L1 ≧ 1/3 X L2 is satisfied.

A catheter according to the present invention is used with its body being percutaneously inserted into the blood vessel.

When an auxiliary blood circulation operation is to be performed during PTCA or IABP heart treatment or during the treatment of acute heart failure, e.g. myocardial infarction, a blood circulation instrument is inserted into the introduction passage of the catheter. The hemostatic valve in the passage allows the blood circulation instrument to be inserted in a liquid tight marker and without involving any blood leakage.

When the catheter is on the blood-drawing side, the associated blood circulation instrument is connected with a blood-drawing line of the extracorporeal circulation circuit so that blood in the patient's body is drawn out to the circuit through the catheter body and the blood circulation instrument.

When the catheter is on the blood pumping-in side, the associated blood circulation instrument is connected with a blood pumping-in line of the circuit so that the extracorporeally circulated blood is pumped into the blood vessel through the blood circulation instrument and the catheter body.

With the catheter according to the present invention which has at least one side holes formed in the catheter body, blood is drawn out or pumped in through not only the opening at the distal end of the body but also the side hole(s) of the body. Therefore, the catheter according to the present invention is able to operate with a small resistance to the flow of blood from the blood vessel to the catheter body or vice versa, hence, with a reduced pressure loss. Thus, the catheter according to the present invention which has a diameter small enough to facilitate percutaneous insertion is capable of reducing the pressure loss, thereby assuring sufficient amounts of drawn-out or pumped-in blood.

The cathether according to the present invention which has said side holes in the body is also such that, when it is used on the side of the blood-drawing line, the negative pressure applied through the line on the catheter body is distributed amoung the opening at the distal end and the side holes. Therefore, it is possible to reduce the risk that the catheter body subjected to the negative pressure may suck the blood vessel wall, to have the openings through which blood flows in, i.e., the opening at the distal end and the side holes, closed.

Because the total opening area and position of the side holes satisfy the above-specified relationship, the amount of the blood drawn out or pumped in through the opening at the distal end of the catheter body can be at least a certain proportion to the total amount of drawn-out blood. Therefore, a certain amount of blood can be positively drawn out or pumped in at the target portion within the blood vessel where the distal end of the catheter body is indwelt, relative to the amount of blood drawn out or pumped in through the side holes.

The catheter of the present invention can be used for extra-corporeal circulation as an introducing instrument including a sheath introducible into a blood vessel and a proximal part communicating with said sheath and having a plurality of introduction passages with hemostatic valves disposed therein, the tubular portion of said connecting instrument is capable of being connected with at least one of said introduction passages and also capable of being inserted in a liquid-tight manner through the hemostatic valve disposed in the connected introduction passages.

An assembly for extracorporeal circulation according to the present invention is used with the sheath of the introducing instrument being percutaneously inserted into the blood vessel.

At this time, a catheter, for example, a PTCA catheter (i.e., a unit having a guide catheter and a balloon catheter guided thereby) is inserted through one of the introduction passages at the proximal part of the introducing instrument. The hemostatic valve in the introduction passage allows the catheter to be inserted into the passage in a liquid tight manner and without the risk of blood leakage. The inserted catheter is introduced through the sheath to the target portion within the blood vessel (the constricted lesion of the coronary artery).

Alternatively, an IABP balloon catheter may be inserted into one of the introduction passages of the introducing instrument. The hemostatic valve in the introduction passage also allows such a catheter to be inserted into the passage in a liquid tight manner and without the risk of blood leakage. The inserted catheter is introduced through the sheath to the target portion of the blood vessel (the aorta).

The introducing instrument may be able to allow a plurality of catheters, such as a PTCA catheter and an IABP catheter, to be individually inserted into the plurality of introduction passages so that these catheters are introduced through the sheath in a parallel manner to the respective target portions of the blood vessel.

During the PTCA or IABP heart treatment operation, when the patient goes into a shocked condition, or when it is necessary to strengthen the heart assisting function of the IABP, the connecting instrument is inserted into another passage of the introducing instrument which is not the passage(s) through which the PTCA and/or IABP catheters are introduced. The hemostatic valve in this other passage enables liquid-tight, blood-leakage free insertion of the connecting instrument. The thus inserted connecting instrument is connected with a blood-transfusion line of an extracorporeal circulation circuit so that, simultaneously with the PTCA or IABP operation, the extracorporeally circulated and oxygenated blood is transferred into the blood vessel through the connecting instrument and the sheath of the introducing instrument.

When the patient is in a critical condition, the connecting instrument may be inserted into one of the introduction passages of the introducing instrument prior to the start of a PTCA or IABP operation so that extracorporeal circulation can be started whenever necessary. In this way, the necessary heart treatment can be performed with a higher level of safety.

In carrying out the present invention, the diameter of a catheter (e.g., a PTCA catheter) inserted into the sheath of the introducing instrument is generally 8F or 9F (F = 1/3 mm). Therefore, in order to assure that, when a catheter is inserted into the sheath and indwelt, the blood from the blood-transfusion line of the extracorporeal circulation circuit is smoothly transferred through the connecting instrument and the sheath portion into the blood vessel, the sheath should preferably have an inner diameter of 3 to 10 mm, more preferably, 5 to 7 mm. If the inner diameter of the sheath is less than 3 mm, it is not possible to assure a sufficient flow passage area between the outer periphery of the catheter and the inner periphery of the sheath. In such cases, it is not possible to achieve the necessary flow of blood being transferred. If the blood circulation is caused under a constant pressure by a blood-transfusion pump in the extracorporeal circulation circuit, the insufficient flow of blood resulting from the insufficient flow passage area leads to increased pressure loss, thereby involving the risk of hemolysis. On the other hand, it the inner diameter of the sheath is more than 10 mm, the percutaneous insertion of the sheath will be difficult.

In carrying out the present invention, if the sheath of the introducing instrument has at least one side hole formed therein, it is possible, when a catheter is inserted into the sheath and indwelt, to assure a sufficient area of opening through which blood flows, thereby enabling the attainment of the necessary blood-transfusion amount.

In carrying out the present invention, if the proximal end of the introducing instrument has a passage capable of communicating with a tube having a cook at one end thereof, this passage may be used to inject a medicine liquid or to collect blood during the heart treatment.

Non limitative examples of the invention will now be described in connection with the annexed drawings.

Fig. 1 is a sectional view of the essential parts of an example of a catheter according to the present invention;

Fig. 2 is a sectional view of the essential parts of an example of a connecting instrument used in combination with the catheter;

Fig. 3 is a sectional view of an example of a blood vessel expander used in combination with the catheter;

Fig. 4 is a sectional view showing a state of use of the catheter;

Fig. 5 is a view schematically showing an indwelt state of the catheter;

Fig. 6 is a sectional view showing another state of use of the catheter;

Fig. 7 is a sectional view of an example of an introducing instrument of an extracorporeal circulation assembly according to the present invention;

Fig. 8 is a sectional view of an example of a connecting instrument of the assembly; and

Fig. 9 is a sectional view of an example of a blood vessel expander used in the assembly.

A catheter assembly 10 basically comprises a catheter 20 and a connecting instrument 30, and additionally comprises a dilator 40.

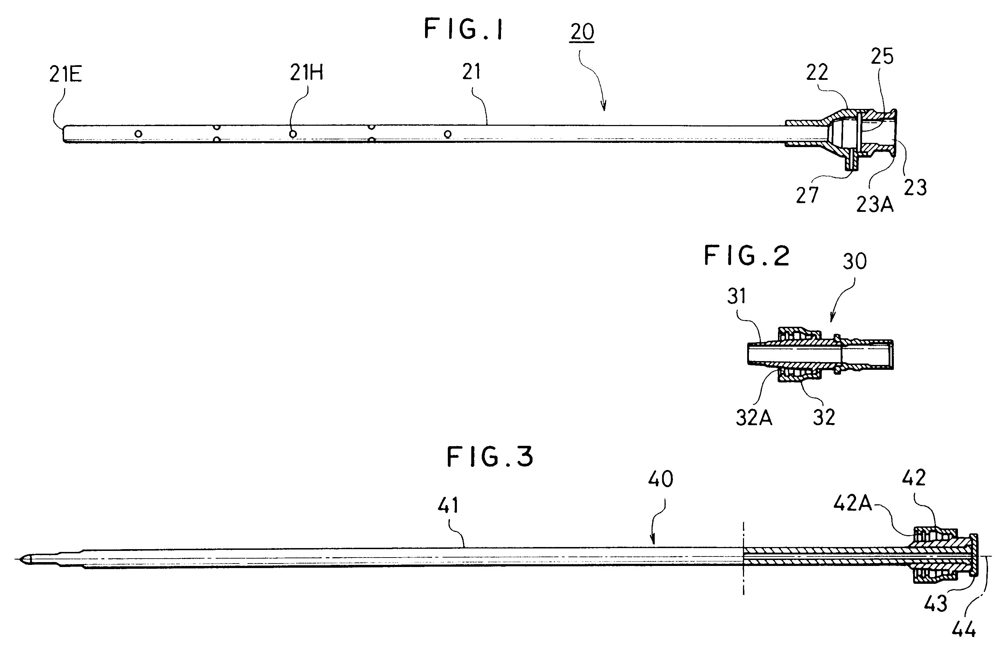

The catheter 20 comprises, as shown in Fig. 1, a body 21 and a proximal end 22.

The body 21 of the catheter 20 is used in the state of being percutaneously inserted into the blood vessel. The catheter body 21 has an opening 21E at the distal end (hereinafter referred to as "the distal-end hole"), and a plurality of side holes 21H.

The proximal end 22 of the catheter 20 is joined to one end portion of the catheter body 21 to communicate therewith, and has an introduction passage 23. The introduction passage 23 has a hemostatic valve 25 which is disposed in an opening portion of the passage 23 and provided to prevent leakage of blood from the catheter body 21 to the outside.

The proximal end 22 also has a sub-passage 27 capable of communicating with a tube having a cock at one end thereof, so as to function as a port which may be used to inject a medicine liquid or collect blood.

The catheter 20 should preferably have an inner diameter of the body 21 which is within the range from 2 to 10 mm in order to facilitate the percutaneous insertion of the catheter body 21. If this inner diameter is less than 2 mm, there is a risk that, during emergency auxiliary circulation, the amount of drawn-out or sent-in blood may fall short of the necessary amount. If the inner diameter exceeds 10 mm, the percutaneous insertion of the catheter 20 will be difficult.

The catheter 20 has the following arrangement. When the opening area of the distal-end hole 21E of the catheter body 21 is expressed as S1, and the total opening area of the side holes 21H is expressed as S2 (S2 = N × Sh if there are N side holes 21E which each have an area of Sh), the relation of 2 × S1 ≧ S2 ≧ 0.5 × S1 is satisfied. In other words, the total opening area S2 of the side holes 21H ranges from 1/2 to 2 times of the opening area S1 of the distal-end hole 21E. If S2 is smaller than 1/2 × S1, when the catheter is on the blood-drawing side, the amount of blood drawn from the region where the side holes open, i.e., from the vena cava, will be insufficient. If S2 is greater than 2 × S1, the amount of the blood drawn from the superior vena cava will be insufficient.

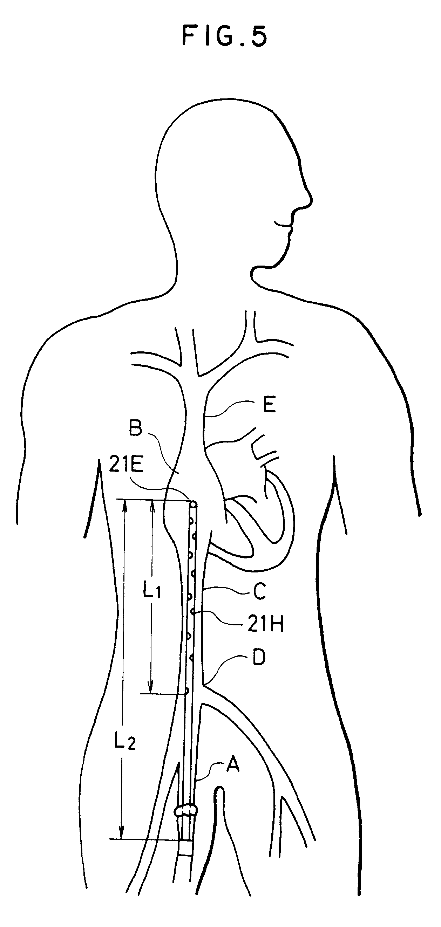

The position at which the side holes 21H are indwelt should preferably be as follows. If the catheter 20 is inserted into the femoral vein A, as shown in Fig. 5, the side holes 21H should be positioned within the inferior vena cava C and in the region from the entrance of the right atrium B to the bifurcation D of the inferior vena cava C (the symbol E designating the superior vena cava). This is because, in this region, blood flows in such a sufficient amount as to assure the drawing of the necessary amount of blood. Therefore, if this position of the side holes 21H relative to the patient's body is translated into their position relative to the catheter body 21 itself, the following can be said. The whole length L2 of the catheter body 21 must correspond to the sum of the first distance from the right atrium to the bifurcation of the inferior vena cava, the second distance from the bifurcation to the position where the catheter is percutaneously inserted into the femoral vein, and an additional length α of the catheter 20 outside the patient's body. In the case where the patient's body has a first distance of 30 to 40 cm and a second distance of 15 to 20 cm (as in the case of an American), the required whole length L2 of the catheter body 21 is 60 cm. In this case, in order to assure the necessary amount of drawn-out blood, the side holes 21H should preferably be positioned occupying a length from the distal end of the catheter body 21 which is 40 cm at most. Therefore, the whole length L2 of the catheter body 21 should satisfy the following relation with the distance L1 from the distal end of the catheter body 21 to the side hole closest to the proximal end 22: 2/3 × L2 ≧ L1. In the case where the patient's body is relatively small (as in the case of Japanese; the patient requiring extacorporeal circulation by the use of the catheter according to the present invention often has a relatively small body), and where the first distance is about 2/3 of the corresponding hemostatic value in above-described case, i.e., about 20 cm, the whole length L2 and the distance L1 should satisfy the relation of L1 ≧ 1/3× L2. Thus, it is preferable that the whole length L2 and the distance L1 should satisfy the relation expressed as follows:

$$2/3 \times L2 \geqq L1 \geqq 1/3 \times L2$$

If the catheter 20 satisfies this relationship it is possible to attain the necessary amount of blood drawn whether the patient's body is large or small.

The catheter body 21 is made of a material such as a fluoroplastic, polyethylene, polypropylene, or a polyester-based elastomer. The proximal end 22 is made of a material such as polyethylene, polypropylene, polyamide, polycarbonate, or polystyrene.

As shown in Fig. 2, the connecting instrument 30 has a tubular portion 31 which can be connected with the introduction passage 23 in the proximal end 22 of the catheter 20, and be inserted in a liquid tight manner through the hemostatic valve 25 in the introduction passage 23. The other end of the tubular portion 31 is connected with the blood drawing-out or pumping-in line of the extracorporeal circulation circuit. The connecting instrument 30 also has a threaded connecting cap 32 on the outer periphery of the tubular portion 31. When a female screw 32A of the connecting cap 32 is threaded onto a male screw 23A provided around the introduction passage 23 of the proximal end 22, the connecting instrument 30 is fixed to the catheter 20.

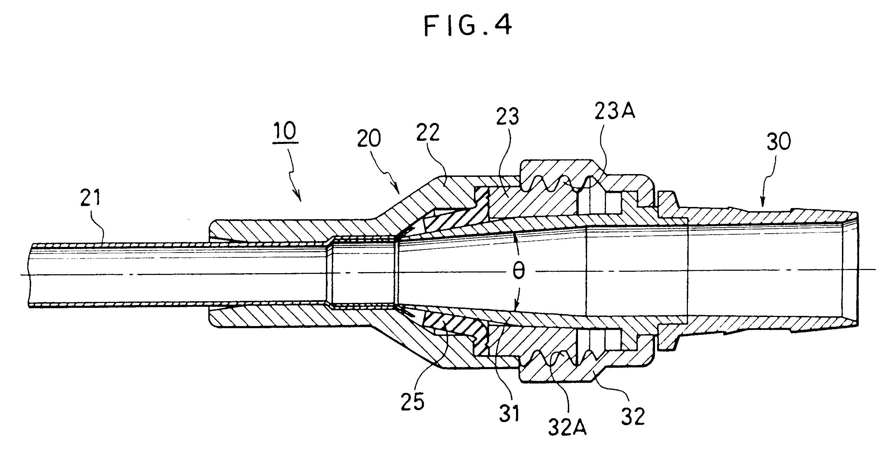

The catheter assembly 10 is such that, when the connecting instrument 30 is connected with the introduction passage 23 of the catheter proximal end 22, as shown in Fig. 4, the distal end of the tubular portion 31 of the connecting instrument 30 is disposed adjacent to the inner surface of the catheter body 21. In this embodiment, that distal end abuts on the inner surface of the catheter body 21.

The inner surface of the tubular portion 31 of the connecting instrument 30 is tapered, more specifically, converged with its diameter decreasing toward the distal end of the portion 31.

The tapering angle $\theta$ (in Fig. 4) at which the inner surface of the tubular portion 31 is tapered should preferably be 5 to 15 degrees.

The tubular portion 31 of the connecting instrument 30 is made of a material such as polycarbonate, a vinyl chloride resin, or polypropylene, while the cap 32 is made of a material such as polyamide, polycarbonate, or a vinyl chloride resin.

The dilator 40 is capable of being inserted into and through the introduction passage 23 of the proximal end 22 of the catheter 20 as well as the body 21 of the catheter 20 so as to lead the catheter body 21 into the blood vessel. As shown in Fig. 3, the dilator 40 comprises a body 41 and a proximal end with a threaded connecting cap 42 provided around the proximal end. When a female screw 42A of the connecting cap 42 is threaded onto the male screw 23A provided around the introduction passage 23 in the catheter proximal end 22 the dilator 40 is integrated with the catheter 20. The body 41 of the dilator 40 is capable of being inserted in a liquid tight manner through the hemostatic valve 25 in the introduction passage 23 of the catheter 20. The dilator 40 also has a hemostatic valve 43 provided at the outer end of the cap 42. The dilator 40 allows the insertion therethrough of a mini guide wire 44 when the wire 44 is passed through the hemostatic valve 43 in a liquid tight manner.

The dilator body 41 is made of a material such as polyethylene, polypropylene, or a polyester-based elastomer, while the connectiong cap 42 is made of a material such as polyamide, polycarbonate, or a vinyl chloride resin.

The catheter 20 of the above-described catheter assembly 10 is inserted into and indwelt in the blood vessel in the following manner:

(1) An indwelling needle having an outer needle member and an inner needle member fit therein is percutaneously inserted into the blood vessel.

(2) The inner needle member is removed. The mini guide wire 44 is passed through the outer needle member to be indwelt in the blood vessel. After the indwelling, the outer needle member is removed.

(3) The catheter 20 and the dilator 40 integrated therewith are inserted into the blood vessel while they are guided by the mini guide wire 44. After the insertion, the mini guide wire 44 and the dilator 40 are removed, thereby indwelling the catheter 20 in the blood vessel.

The catheter 20 indwelt in the blood vessel is used in the following manner:

① When auxiliary blood circulation is to be performed during PTCA or IABP heart treatment or during the treatment of acute myocardial infarction or heart failure, the connecting instrument 30 is inserted into the introduction passage 23 of the catheter 20. By virtue of the hemostatic valve 25 of the introduction passage 23, the connecting instrument 30 is inserted into the passage 23 in a liquid tight manner and without the risk of blood leakage.

② The connecting instrument 30 of the catheter assembly 10 on the blood-drawing side is connected with the blood-drawing line of the extracorporeal circulation circuit so that blood is drawn from the patient's body through the catheter body 21 and the tubular portion 31 of the connecting instrument 30.

③ The connecting instrument 30 of the catheter assembly 10 on the blood pumping-in side is connected with the blood pumping-in line of the extracorporeal circulation circuit so that the extracorporeally circulated blood is pumped into the blood vessel through the tubular portion 31 of the connecting instrument 30 and the catheter body 21.

The catheter 20 according to the present invention can be percutaneously inserted into the femoral vein in such a manner that the distal-end hole 21E of the catheter body 21 is positioned in the vicinity of the right atrium, and can be connected with the blood-drawing line of the extracorporeal circulation circuit so as to draw blood from the right atrium and the venae cavae. At this time, the position of the side holes 21H of the catheter body 21 is such that the holes 21H are distributed from the vicinity of the bifurcation of the inferior vena cava to the vicinity of the right atrium.

The catheter 20 provides the following advantages (1) to (3):

(1) The catheter 20 allows blood to be drawn out or pumped in through not only the distal-end hole 21E of the catheter body 21 but also the side holes 21H thereof. Therefore, the catheter 20 according to the present invention is capable of reducing the resistance to the flow of blood from the blood vessel to the catheter body 21 or vice veasa, thereby reducing the pressure loss. Thus the catheter 20, of which the diameter is small enough to facilitate the percutaneous insertion of the catheter 20, is capable of reducing the pressure loss, hence, of assuring sufficient amount of drawn-out or pumped-in blood.

(2) When the catheter 20 is used on the side of the blood-drawing line, by virtue of the provision of the side holes 21H in the catheter body 21, the negative pressure applied through the blood-drawing line to the body 21 is distributed among the distal-end hole 21E and the side holes 21H of the catheter body 21, thereby reducing the risk that the negative pressure may cause the blood vessel wall to be sucked by the body 21, to close the inlets (the distal-end hole 21E and the side holes 21H) through which blood flows in.

(3) It is possible to assure that the amount of blood drawn out or pumped in through the distal-end hole 21E is at least at a certain proportion to the total amount of drawn-out blood. Therefore, a certain amount of blood can be positively drawn out or pumped in at the target portion within the blood vessel where the leading end of the catheter body is indwelt, relative to the amount of blood drawn out or pumped in through the side holes.

This advantage will be understood clearly by considering the following case. When the body 21 of the catheter 20 is percutaneously inserted into, for instance, the femoral vein with the distal-end hole 21E being positioned in the right atrium, as described above, so as to draw blood, if all of the amount of blood to be extracorporeally circulated is drawn through the side holes 21H, this makes it impossible for blood from the superior vena cava to be circulated in the extracorporeal circuit, thereby, resulting in a very dangerous state. In contrast, the catheter 20 according to the present invention is such that, as described in (3), it does not cause all of the circulation amount to be drain through the side holes 21H, but causes a large amount of blood to be drawn through the distal-end hole 21E. Thus, the catheter 20 enables blood which is circulating from the superior vena cava to the right atrium to be drawn through the distal-end hole 21E and extracorporeally circulated.

Experiments were conducted on a specific sample of the catheter 20, in which the length of the body 21 was 20 cm, the inner diameter was 5.2 mm, the number of side holes 21H was six, the diameter of the side holes 21H was 2.1 mm, and the side holes 21H were formed at intervals of 5 cm within a region extending through 30 cm from the distal-end of the body 21. The opening area S1 of the distal-end hole 21E was 21.23 mm$^2$, and the total opening area of the side holes 21H was 20.76 mm$^2$, thereby satisfying the relation of $2S1 \geqq S2 \geqq 0{,}5S1$. As a result, the catheter 20, which had a diameter small enough to facilitate percutaneous insertion at the time of auxiliary blood circulation, was found to be capable of assuring a sufficient amount of drawn-out or pumped-in blood.

According to the above-described embodiment, since the catheter 20 has the sub-passage 27 at the proximal end 22, it is possible to use the passage 27 to perform the injection of a medicine liquid or the collection of blood simultaneously with heart treatment or the like.

In carring out the present invention, the introduction passage of the proximal end of the catheter may alloy the insertion of an insertion body other than the connecting instrument connected with the blood-drawing line or the blood pumping-in line of the extracorporeal circulation circuit. For instance, a catheter for heart treatment (e.g,, a unit having a guide catheter and a balloon catheter guided thereby), or a connecting instrument connected with a medicine liquid supply line may be inserted into the introduction passage.

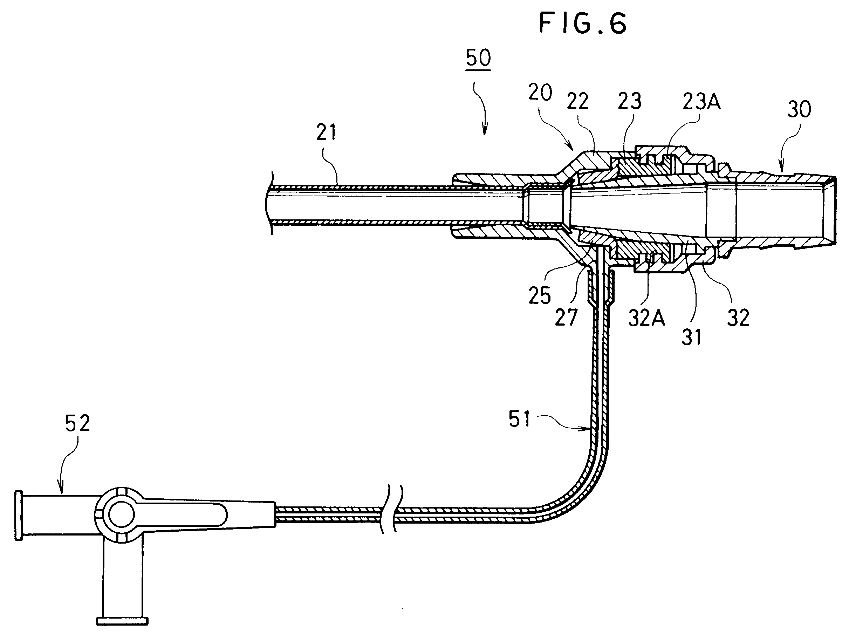

A catheter assembly 50, shown in Fig 6, is a modification of the above-described catheter assembly 10. Similarly to the catheter assembly 10, the catheter assembly 50 basically comprises a catheter 20 and a connecting instrument 30, and additionally comprises a dilator 40.

The substantial difference of the catheter assembly 50 from the catheter assembly 10 is that when the connecting instrument 30 is connected to the introduction passage 23 of the catheter 20, the distal-end of the tubular portion 31 of the connecting instrument 30 does not abut on the inner surface of the body 21 of the catheter 20, but is disposed close to the inner surface with a certain gap.

Therefore, with the catheter assembly 50, when the sub-passage 27 in the proximal end 22 of the catheter 20 is connected through a side tube 51 and a three-way cock 52 to a pressure transducer (not shown) so as to measure the blood pressure, the blood pressure introduced by the catheter body 21 can be applied to the sub-passage 27 through the gap between the inner surface of the body 21 and the distal-end of the tubular portion 31, thus without any substantial resistance. This enables highly precise pressure measurement.

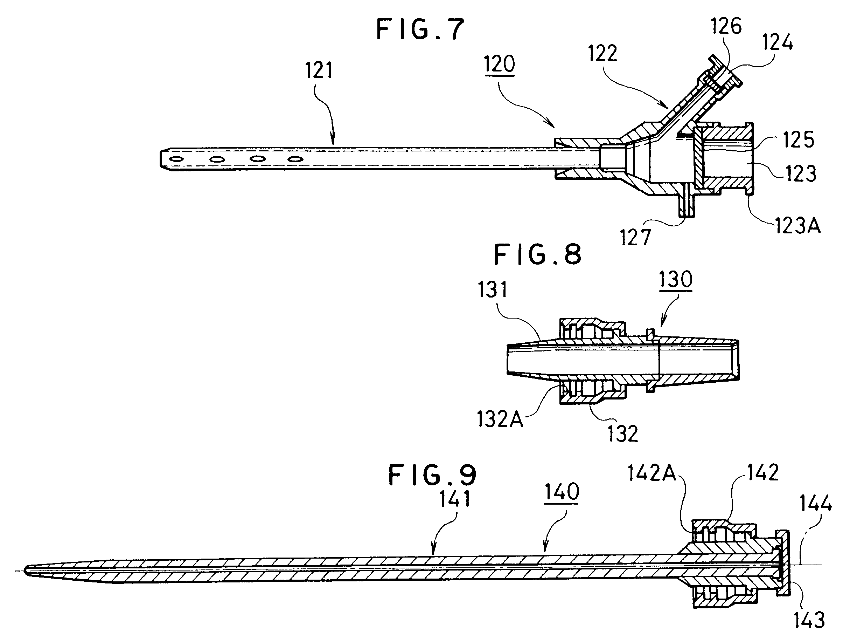

In a further embodiment illustrated on figures 7 to 9, an assembly for extracorporeal circulation basically comprises an introducing instrument 120 and a connecting instrument 130, and additionally comprises a dilator 140.

The introducing instrument 120 comprises, as shown in Fig. 7, a sheath 121 and a proximal part 122.

The sheath 121 is used in the state of being percutaneously inserted into the blood vessel. The sheath 121 includes a plurality of side holes 121H.

The proximal part 122 of the introducing instrument 120 is jointed to one end portion of the sheath 121 to communicate therewith, and has a first introduction passage 123 as well as a second introduction passage 124. The introduction passages 123 and 124 have hemostatic valves 125 and 126, respectively, which are disposed in opening portion of the passages 123 and 124, and provided to prevent leakage of blood from the sheath 121 to the outside.

The proximal part 122 also has a sub-passage 127 capable of communicating with a tube having a cock at one end thereof, so as to function as a port which may be used to inject a medicine liquid or collect blood.

The sheath 121 is made of a material such as a fluoroplastic, polyethylene, or a polyester-based elastomer. The proximal part 122 is made of a material such as polyethylene, polypropylene, polyamide, polycarbonate, or polystyrene.

As shown in Fig. 8, the connecting instrument 130 has a tubular portion 131 which can communicate with the first introduction passage 123 in the proximal part 122 of the introducing instrument 120, and be inserted in a liquid tight manner through the hemostatic valve 125 in the introduction passage 123. The other end of the tubular portion 131 is connected with the blood-transfusion line of the extracorporeal circulation circuit. The connecting instrument 130 also has a threaded connecting cap 132 on the outer periphery of the tubular portion 131. When a female screw 132A of the connecting cap 132 is threaded onto a male screw 123A provided around the first introduction passage 123 of the proximal part 122, the connecting instrument 130 is fixed to the introducing instrument 120.

The tubular portion 131 of the connecting instrument 130 is made of a material such as polycarbonate, a vinyl chloride resin, or polypropylene, while the cap 132 is made of a material such as polyamide, polycarbonate, or a vinyl chloride resin.

The dilator 140 is capable of being inserted into and through the first introduction passage 123 of the proximal part 122 of the introducing instrument 120 as well as the sheath 121 of the instrument 120 so as to lead the sheath 121 into the blood vessel. As shown in Fig. 9, the dilator 140 comprises a body 141 and a proximal part with a threaded connecting cap 142 provided around the proximal part. When a female screw 142A of the connecting cap 142 is threaded onto the male screw 123A provided around the first introduction passage 123 in the proximal part 122, the dilator 140 is integrated with the introducing instrument 120. The body 141 of the dilator 140 is capable of being inserted in a liquid tight manner through the hemostatic valve 125 in the introduction passage 123 of the proximal part 122. The dilator 140 also has a hemostatic valve 143 provided at the outer end of the cap 142. The dilator 140 allows the insertion therethrough of a mini guide wire 144 when the wire 144 is passed through the hemostatic valve 143 in a liquid tight manner.

The dilator 140 is made of a material such as polyethylene, polypropylene, or a polyester-based elastomer, while the connecting cap 142 is made of a material such as polyamide, polycarbonate, or a vinyl chloride resin.

The introduction instrument 120 of the above-described extracorporeal circulation assembly is inserted into and indwelt in the blood vessel in the following manner:

(1) An indwelling needle having an outer needle member and an inner needle member fit therein is percutaneously inserted into the blood vessel.

(2) The inner needle member is removed. The mini guide wire 144 is passed through the outer needle member to be indwelt in the blood vessel. After the indwelling, the outer needle member is removed.

(3) The introducing instrument 120 and the dilator 140 integrate, therewith are inserted into the blood vessel while they are guided by the mini guide wire 144. After the insertion, the mini guide wire 144 and the dilator 140 are removed, thereby indwelling the introducing instrument 120 in the blood vessel.

The introducing instrument 120 indwelt in the blood vessel is used in the following manner:

(a) First an insertion body such as a PCTA catheter (a unit having a guide catheter and a balloon catheter guided theterby) is inserted into the second introduction passage 124 in the proximal part 122 of the introducing instrument 120 while this catheter is guided by a guide wire already passed through the second introduction passage 124. By virtue of the hemostatic valve 126 of the second introduction passage 124, the catheter is inserted into the introduction passage 124 in a liquid tight manner and without the risk of blood leakage. The inserted catheter is introduced, through the sheath 121, to the target portion in the blood vessel (the lesion in the coronary artery).

Alternatively, an IABP balloon catheter may be inserted into the second introduction passage 124 of the introducing instrument 120 while this catheter is guided by a guide wire already passed through the second introduction passage 124. Also in this case, the hemostatic valve 126 of the second introduction passage 124 enables the liquid-tight, blood-leakage free insertion of the catheter into the introduction passage 124. The inserted catheter is introduced, through the sheath 121, to the target portion in the blood vessel (the proximal part portion of the aorta).

(b) During the PTCA or IABP heart treatment operation, when the patient goes into a shocked condition, or when it is necessary to strengthen the heart assisting function of the IABP, the connecting instrument 130 is inserted into the first introduction passage 123 of the introducing instrument 120. By virtue of the hemostatic valve 125 of the first introduction pass age 123, the connecting instrument 130 is inserted into the introduction passage 123 in a liquid tight manner and without the risk of blood leakage. The inserted connecting instrument 130 is connected with a blood-transfusion line of an extracorporeal circulation circuit, so that, simultaneously with the PTCA or IABP operation, the extracorporeally circulated and oxygenated blood is transferred to the blood vessel through the connecting instrument 130 and the sheath 121.

When the patient is in a critical condition, the connecting instrument 130 may be inserted into the first introduction passage 123 of the introducing instrument 120 prior to the start of the PTCA or IABP operation so that extracorporeal circulation can be started whenever necessary. In this way, the necessary heart treatment can be performed with a higher level of safety.

In the above-described enbodiment, the diameter of a catheter, such as PTCA catheter, inserted into the sheath 121 of the introducing instrument 120 is generally 8F or 9F. Therefore, in order to assure that, when a catheter is inserted into the sheath 121 and indwelt, the blood from the blood-transfusion line of the extracorporeal circulation circuit is smoothty transferred through the connecting instrument 130 and the sheath 121 into the blood vessel, the inner diameter of the sheath 121 should preferably be 3 to 10 mm, more preferably 5 to 7 mm, for the reasons stated before in the summary section.

In the above-described embodiment, since the sheath 121 of the introducing instrument 120 has the side holes 121H, it is possible, when a catheter is inserted into the sheath 121 and indwelt, to assure a sufficient area of opening through which blood flows, thereby enabling the attainment of the necessary blood-transfusion amount.

Also in the above-described enbodiment, since the proximal part 122 of the introducing instrument 120 has the sub-passage 127, it is possible to use the passage 127 to perform the injection of a medicine liquid or the collection of blood simultaneously with heart treatment or the like.

In carrying out the present invention, the proximal part of the introducing instrument may have three or more introduction passages.

In carrying out the present invention, a plurality of connecting instruments may be individually connected to two or more introduction passages of the proximal part of the introducing instrument.

In carrying out the present invention, the introduction passages of the proximal part of the introducing instrument may allow the insertion of insertion bodies other than the catheter and the connecting instrument connected with the blood-transfusion line of the extracorporeal circulation circuit. For instance, a connecting instrument connected with a medicine liquid supply line may be inserted into the introduction passage.

As has been described above, according to the present invention, there is provided a catheter that has a diameter small enough to facilitate percutaneous insertion at the time of auxiliary blood circulation but is capable of assuring a sufficient amount of drawn-out or pumped-in blood.

According to the present invention, there is also provided an assembly for extracorporeal circulation that enable extracorporeal circulation to be effected simultaneously with the execution of heart treatment.

**Claims**

1. A catheter assembly comprising a catheter (20) and a connecting instrument (30) ; the catheter (20) having a hollow body (21) introducible into a blood vessel and provided with an opening (21E) at a distal end thereof and a plurality of side holes (21H), a proximal end communicating with the hollow inside of said body (21) and having an introduction passage (23) with a hemostatic valve (25) disposed therein ; characterized in that the area (S1) of the opening (21E) at the distal end of said body (21) is such as compared to the total opening area (S2) of said plurality of side holes (21H) that the relation $2 \times S1 \geq S2 \geq 0.5 \times S1$ is satisfied ; and the connecting instrument (30) has a tubular portion (31) adapted to be inserted in said introduction passage (23) in a liquid-tight manner through the hemostatic valve (25) the inner surface of the tubular portion (31) being tapered and the distal end of the tubular portion (31) being disposed close or in abutting relation to the inner surface of the catheter body (21).

2. A catheter according to claim 1, wherein the distance (L1) from the distal end of said body to the side hole closest to said proximal end is such as compared to the whole length (L2) of said body, that the relation of $2/3 \times L2 \geq L1 \geq 1/3 \times L2$ is satisfied.

3. A catheter assembly according to claim 1 or 2, wherein the inner diameter of the catheter body (21) is within the range from 2 to 10 mm.

4. Catheter assembly according to anyone of claims 1 to 3, wherein the tapering angle of the tubular portion (31) of the connecting instrument is of 5 to 15°.

5. Catheter assembly according to anyone of claims 1 to 4 further comprising a dilator (40) capable of being inserted into end through said introduction passage (23) of the catheter (20) through the hemostatic valve (25).

6. Catheter assembly according to anyone of claims 1 to 5, wherein the proximal end of the catheter (20) is further provided with a sub-passage (27) which may be connected to a side tube.

7. Catheter assembly of any preceding claim for extra-corporeal circulation, characterized in that the catheter includes a sheath (121) introducible into a blood vessel and a proximal part (122) communicating with said sheath (121) and having a plurality of introduction passages (123, 124) with hemostatic valves (125, 126) disposed therein, said tubular portion (131) of said connecting instrument (130) being capable of being connected with at least one of said introduction passages and capable of being inserted in a liquid-tight manner through the hemostatic valve disposed in the connected introduction passage.

## Patentansprüche

1. Katheterinstrumentarium, mit: einem Katheter (20) und einer Verbindungsvorrichtung (30), wobei der Katheter (20) ein Hohlteil (21) hat, das in ein Blutgefäß einführbar ist, und an seinem distalen Endabschnitt mit einer Öffnung (21E) und einer Vielzahl von Seitenlöchern (21H) versehen ist, wobei ein proximaler Endabschnitt mit der hohlen Innenseite des Teils (21) in Verbindung steht, und einen Einleitungskanal mit einem darin angeordneten hämostatischen Ventil (25) hat, **dadurch gekennzeichnet, daß** die Fläche (S1) der Öffnung (21E) an dem distalen Endabschnitt des Teils (21) im Vergleich zu der Gesamtöffnungsfläche (S2) der Vielzahl von Seitenlöchern (21H) derart ist, daß die Beziehung $2 \times S1 \geq S2 \geq 0.5 \times S1$ erfüllt ist, und die Verbindungsvorrichtung (30) einen röhrenförmigen Abschnitt (31) hat, der dazu angepaßt ist, durch das hämostatische Ventil (25) auf eine flüssigkeitsdichte Weise in den Einleitungskanal (23) eingeführt zu werden, wobei die innere Fläche des röhrenförmigen Abschnitts (31) kegelförmig ist, und der distale Endabschnitt des röhrenförmigen Abschnitts (31) nahe der oder in Anlage-Beziehung zu der inneren Fläche des Katheterteils (21) angeordnet ist.

2. Katheter gemäß Anspruch 1, wobei der Abstand (L1) von dem distalen Endabschnitt des Teils zu dem Seitenloch, das dem proximalen Endabschnitt am nächsten ist, im Vergleich zu der gesamten Länge (L2) des Teils derart ist, daß die Beziehung $2/3 \times L2 \geq L1 \geq 1/3 \times L2$ erfüllt ist.

3. Katheterinstrumentarium gemäß Anspruch 1 oder 2, wobei der innere Durchmesser des Katheterteils (21) innerhalb des Bereichs von 2 bis 10 mm ist.

4. Katheterinstrumentarium gemäß einem der Ansprüche 1 bis 3, wobei der Kegelwinkel des röhrenförmigen Abschnitts (31) der Verbindungsvorrichtung 5 bis 15° ist.

5. Katheterinstrumentarium gemäß einem der Ansprüche 1 bis 4, das ferner einen Dilator (40) aufweist, der durch das hämostatische Ventil (25) in und durch den Einleitungskanal (23) des Katheters (20) einführbar ist.

6. Katheterinstrumentarium gemäß einem der Ansprüche 1 bis 5, wobei der proximale Endabschnitt des Katheters (20) ferner mit einem Nebenkanal (27) versehen ist, der mit einer Seitenröhre verbunden werden kann.

7. Katheterinstrumentarium zur extrakorporalen Zirkulation gemäß einem vorangehenden Anspruch, **dadurch gekennzeichnet, daß** der Katheter eine in ein Blutgefäß einleitbare Hülse (121) und ein proximales Teil (122) aufweist, das mit der Hülse (121) in Verbindung steht, und eine Vielzahl von Einleitungskanälen (123, 124) mit darin angeordneten hämostatischen Ventilen (125, 126) hat, wobei der röhrenförmige Abschnitt (131) der Verbindungsvorrichtung (130) mit mindestens einem der Einleitungskanäle verbindbar ist, und durch das in dem verbundenen Einleitungskanal angeordnete hämostatische Ventil auf eine flüssigkeitsdichte Weise einführbar ist.

## Revendications

1. Ensemble de cathéter comprenant un cathéter (20) et un instrument de raccordement (30), le cathéter (20) ayant un corps creux (21) qui peut être introduit dans un vaisseau sanguin et qui est muni d'une ouverture (21E) en son extrémité distale et d'une pluralité de trous latéraux (21H), une extrémité proximale qui communique avec l'intérieur creux dudit corps (21) et qui comporte un passage d'introduction (23) dans lequel est placée une valve hémostatique (25),

caractérisé en ce que la superficie (S1) de l'ouverture (21E) se trouvant à l'extrémité distale dudit corps (21) est telle, comparée à la superficie totale d'ouverture (S2) de ladite pluralité de trous latéraux (21H), que la relation $2 \times S1 \geq S2 \geq 0,5 \times S1$ est satisfaite, et l'instrument de raccordement (30) comprend une partie tubulaire (31) pouvant être introduite dans Ledit passage d'introduction (23) d'une manière étanche aux liquides par l'intermédiaire de la valve hémostatique (25), la surface intérieure de la partie tubulaire (31) étant conique et l'extrémité distale de la partie tubulaire (31) étant placée près de la surface intérieure du corps (21) de cathéter ou en contact avec elle.

2. Cathéter selon la revendication 1, dans lequel la distance (L1) entre l'extrémité distale dudit corps et le trou latéral le plus proche de ladite extrémité proximale est telle, comparée à la longueur totale (L2) dudit corps, que la relation $2/3 \times L2 \geq L1 \geq 1/3 \times L2$ est satisfaite.

3. Ensemble de cathéter selon la revendication 1 ou 2, dans lequel le diamètre intérieur du corps (21) de cathéter se situe dans la plage allant de 2 à 10 mm.

4. Ensemble de cathéter selon l'une quelconque des revendications 1 à 3, dans lequel l'angle de conicité de la partie tubulaire (31) de l'instrument de raccordement vaut de 5 à 15°.

5. Ensemble de cathéter selon l'une quelconque des revendications 1 à 4, comprenant en outre un dilatateur (40) pouvant être introduit dans et à travers ledit passage d'introduction (23) dudit cathéter (20) par l'intermédiaire de la valve hémostatique (25).

6. Ensemble de cathéter selon l'une quelconque des revendications 1 à 5, dans lequel l'extrémité proximale du cathéter (20) est en outre pourvue d'un passage auxiliaire (27) qui peut être raccordé à un tube latéral.

7. Ensemble de cathéter selon l'une quelconque des revendications précédentes, destiné à une circulation extracorporelle, caractérisé en ce que le cathéter comprend une gaine (121) pouvant être introduite dans un vaisseau sanguin et une pièce proximale (122) qui communique avec ladite gaine (121) et qui présente une pluralité de passages d'introduction (123, 124) dans lesquels sont placées des valves hémostatiques (125, 126), ladite partie tubulaire (131) dudit instrument de raccordement (130) pouvant être raccordée à l'un au moins desdits passages d'introduction et pouvant être introduite d'une manière étanche aux liquides par l'intermédiaire de la valve hémostatique placée dans le passage d'introduction raccordé.

FIG.1

21E  21H  21  20  22  25

27  23A  23

FIG.2  30

31

32A  32

FIG.3

41  40  42  42A

43  44

EP 0 411 605 B1

# FIG.4

EP 0 411 605 B1

# FIG.5

# FIG.6

EP 0 411 605 B1

FIG.7

120

121

122

124

126

125

123

123A

127

FIG.8

130

131

132

132A

FIG.9

140

141

142

142A

144

143